# FASCICULE DE BREVET EUROPEEN

(11) **EP 1 195 157 B1**
(45) Date de publication et mention de la délivrance du brevet: **02.11.2005**
(21) Numéro de dépôt: 01402454.1
(22) Date de dépôt: 25.09.2001
(51) Int. Cl.: A61K 7/48, A61K 7/021

(54) **Composition de maquillage**
Zusammensetzung für das Schminken
Make-up composition

(30) Priorité: 09.10.2000 FR 0012882
(43) Date de publication de la demande: 10.04.2002
(73) Titulaire: L'OREAL, 75008 Paris (FR)
(72) Inventeur: Bara, Isabelle, 75013 Paris (FR)
(74) Mandataire: Kromer, Christophe

(56) Documents cités:
- US-A- 5 306 487
- DATABASE CHEMICAL ABSTRACTS [en ligne] STN; access number 132: 339 081, XP002170501 & JP 2000 143480 A (KOSEI CO.) 23 mai 2000 (2000-05-23)

## Description

L'invention a pour objet une composition cosmétique comprenant un polymère filmogène et des particules de polymère absorbant d'eau. L'invention a aussi pour objet un procédé de maquillage des matières kératiniques. La composition et le procédé de maquillage selon l'invention sont plus particulièrement destinés au maquillage des matières kératiniques comme la peau, y compris les lèvres et le cuir chevelu, et les phanères tels que les ongles, les cheveux, les cils et les sourcils d'êtres humains.

Cette composition peut se présenter sous la forme de maquillage du corps, de la peau du visage, des oreilles (et notamment le lobe), de produit de maquillage des lèvres, de produit de maquillage des cils, de produit de maquillage des ongles, de produit de maquillage des cheveux.

Les produits de maquillage sont couramment employés pour apporter de la couleur, mettre en valeur certaines parties de la peau ou des phanères, ou bien encore procurer un aspect brillant, mat, satiné à la peau ou aux phanères. Ces produits sont habituellement appliqués sous la forme d'une mince couche uniforme.

Pour conférer au produit de maquillage une bonne tenue sur la peau ou les phanères, il est connu d'utiliser des polymères filmogènes en dispersion aqueuse. Par exemple, le brevet US-A-3639572 décrit une composition de maquillage de la peau comprenant une dispersion aqueuse de polymère tels que les esters polyacryliques. Cette composition laisse après séchage un film de bonne tenue sur la peau. Le brevet US-A-4158053 décrit des vernis-à-ongles comprenant une dispersion aqueuse de polymère acrylique formant un film adhérant sur les ongles.

Avec l'évolution de la mode, les consommateurs, plus exigeants, recherchent de nouveaux produits de maquillage permettant d'obtenir des effets de maquillage originaux ou particuliers, et notamment conférant des transformations visibles du visage ou du corps maquillés.

Or, les compositions de maquillage actuellement disponibles sur le marché ne permettent que l'obtention d'un film uniforme et lisse et pas de maquillage en relief. Un besoin subsiste donc de disposer de produit de maquillage dont l'application sur la peau ou les phanères permettant d'obtenir un effet de maquillage différent des films uniformes et lisses actuellement obtenus avec les produits disponibles sur le marché.

La présente invention a donc pour but de proposer une composition de maquillage permettant d'obtenir un maquillage tridimensionnel, ou en relief, sur la peau et sur les phanères.

La demanderesse a constaté qu'un nouveau type de maquillage des matières kératiniques pouvait être obtenu à l'aide d'une composition comprenant un polymère filmogène et des particules de polymère superabsorbant d'eau. La composition selon l'invention conduit ainsi à la formation d'un dépôt en relief sur les matières kératiniques. Le dépôt en relief est obtenu lors de l'application de la composition selon l'invention sur les matières kératiniques. Ce dépôt comprend des particules individualisées de polymère superabsorbant ressemblant à des cristaux donnant l'illusion de bijoux. On peut donc ainsi créer des bijoux sur la peau et les phanères sans utiliser des pierres précieuses qui sont couteuses. En outre, la composition selon l'invention appliquée sur les cils permet d'épaissir ces derniers.

De façon plus précise, la présente invention a pour objet une composition cosmétique comprenant, dans un milieu cosmétiquement acceptable, un polymère filmogène, caractérisée par le fait qu'elle comprend un polymère superabsorbant d'eau sous forme de particules gonflée d'eau ayant une taille moyenne au moins égale à 0,5 mm.

L'invention a aussi pour objet un procédé cosmétique de maquillage des matières kératiniques comprenant l'application sur lesdites matières kératiniques d'une composition telle que décrite précédemment.

L'invention a encore pour objet l'utilisation d'une composition telle que définie précédemment pour obtenir un maquillage des matières kératiniques présentant l'aspect de cristaux et/ou de bijou.

L'invention a également pour objet l'utilisation, dans une composition cosmétique, d'un polymère filmogène et de particules d'un polymère superabsorbant d'eau sous forme de particules gonflée d'eau ayant une taille moyenne au moins égale à 0,5 mm, pour obtenir un maquillage des matières kératiniques présentant l'aspect de cristaux et/ou de bijou.

L'invention a encore pour objet l'utilisation d'une composition telle que définie précédemment pour épaissir les cils.

L'invention a également pour objet l'utilisation, dans une composition cosmétique, d'un polymère filmogène et de particules d'un polymère superabsorbant d'eau sous forme de particules gonflée d'eau ayant une taille moyenne au moins égale à 0,5 mm, pour épaissir les cils.

Selon la présente demande, on entend par "polymère superabsorbant d'eau", un polymère apte à son état sec d'absorber spontanément au moins 20 fois son poids de fluide aqueux, en particulier d'eau et notamment d'eau distillée. De tels polymères super absorbants sont décrits dans l'ouvrage "Absorbent polymer technology, Studies in polymer science 8" de L. BRANNON-PAPPAS et R. HARLAND, édition Elsevier, 1990.
Ces polymères ont une grande capacité d'absorption et de rétention de l'eau et de fluides aqueux. Après absorption du liquide aqueux, les particules du polymère ainsi imbibées de fluide aqueux restent insolubles dans le fluide aqueux et conservent ainsi leur état particulaire individualisé.

Par absorption spontannée, on entend un temps d'absorption allant jusqu'à 30 minutes.
Le polymère superabsorbant peut avoir une capacité d'absorption d'eau allant de 20 à 2000 fois son propre poids (soit 20 g à 2000 g d'eau absorbée par gramme de polymère absorbant), de préférence de 30 à 1500 fois, et mieux de 50 à 1000 fois. Ces caractéristiques d'absorption d'eau sont définies aux condition normales de température (25 °C) et de pression (760 mm Hg soit 100000 Pa) et pour de l'eau distillée.

La taille des particules de polymère superabsorbant mentionnée est la taille de ces particules après gonflement du polymère dans l'eau. Les particules de polymère superabsorbant, gonflées d'eau, ont une taille moyenne supérieure à 0,5 mm (notammant allant de 0,5 mm à 20 mm), de préférence de 1 mm à 15 mm, et mieux de 3 mm à 10 mm. On entend par taille des particules la plus grande dimension de ces particules.

Comme exemple de polymères superabsorbants, on peut citer :
- les polymères résultants de la polymérisation avec réticulation partielle de monomères à insaturation éthylénique hydrosolubles, comme les polymères acryliques ou vinyliques, en particulier les polyacrylates réticulés et neutralisés,
- les amidons greffés polyacrylates,
- les copolymères acrylamide/acide acrylique, notamment sous forme de sels de sodium,
- les amidons greffés acrylamide/acide acrylique, notamment sous forme de sels de sodium ou de potassium,
- les copolymères isobutylène/anhydride maléique,
- les sels de sodium ou de potassium de carboxyméthyl cellulose,
- les sels d'acide polyaspartique réticulés,
- les associations chitosane/polyvinyl pyrrolidone, chitosane /polyéthylène imine,

Comme polymère superabsorbant, on peut utiliser :
- les polyacrylates de sodium ou de potassium réticulés vendus sous les dénominations "SALSORB CL10", "SALSORB CL 20", "FSA type 101", "FSA type 102" par la société ALLIED COLLOIDS, "ARASORB S-310" par la société ARAKAWA Chemical, "ASAP 2000", "ARIDALL 1460" par la société CHEMDAL, "KI-GEL 201-K" de la société Siber Hegner, "AQUALIC CA W3", "AQUALIC CA W7", "AQUALIC CA W10" par la société NIPPON SHOKUBAÏ, "AQUA KEEP D 50", "AQUA KEEP D 60", "AQUA KEEP D 65", "AQUA KEEP S 30", "AQUA KEEP S 35", "AQUA KEEP S 45", "AQUA KEEP A1 M1", "AQUA KEEP A1 M3" par la société ATOCHEM, "SANWET IM-5000D" de la société HOECHST CELANESE ;
- les polyacrylates greffés d'amidon vendus sous les dénomination "SANWET IM-100", "SANWET IM-3900", "SANWET IM-5000S" par la société HOECHST ;
- les copolymères acrylamide/acide acrylique greffés d'amidon sous forme de sel de sodium ou de potassium vendus sous les dénominations "WATERLOCK A-100", "WATERLOCK A-200", "WATERLOCK D-200", "WATERLOCK B-204" par la société GRAIN PROCESSING CORPORATION ;
- les copolymères acrylamide/acide acrylique sous forme de sel de sodium vendu sous la dénomination "WATERLOCK G-400" par la société GRAIN PROCESSING CORPORATION ;
- le copolymère isobutylène/anhydride maléique vendu sous la dénomination "KI GEL-201 K" ;
- la carboxyméthyl cellulose vendue sous les dénominations "AQUASORB A250" par la société AQUALON ;
   - - les associations chitosane/polyvinyl pyrrolidone vendue sous la dénomination "HYDROGEL AQUATRIX 2", chitosane /polyéthylène imine vendue sous la dénomination "HYDROGEL AQUATRIX 3" par la société HYDROMER.

Le polymère superabsorbant peut être présent dans la composition selon l'invention en une teneur allant de 0,1 à 50 % en poids, par rapport au poids total de la composition, et de préférence de 0,5 à 40 % en poids, et mieux de 1 à 30 % en poids.

La composition selon l'invention contient un polymère filmogène (appelé polymère) pour permettre une bonne cohésion et un bon maintien des particules de polymère superabsorbant sur les matières kératiniques. Du fait de la propriété filmogène du polymère, ce polymère est différent du second polymère superabsorbant qui n'est pas filmogène.

Dans la présente demande, on entend par "polymère filmogène", un polymère apte à former à lui seul ou en présence d'un agent auxiliaire de filmification, un film continu et adhérent sur un support, notamment sur les matières kératiniques.

On utilise de préférence un polymère filmogène sous forme de particules en dispersion aqueuse, connu généralement sous le nom de latex ou pseudolatex.

Parmi les polymères filmogènes utilisables dans la composition de la présente invention, on peut citer les polymères synthétiques, de type radicalaire ou de type polycondensat, les polymères d'origine naturelle, et leurs mélanges.

Par polymère filmogène radicalaire, on entend un polymère obtenu par polymérisation de monomères à insaturation notamment éthylénique, chaque monomère étant susceptible de s'homopolymériser (à l'inverse des polycondensats).
Les polymères filmogènes de type radicalaires peuvent être notamment des polymères, ou des copolymères, vinyliques, notamment des polymères acryliques.

Les polymères filmogènes vinyliques peuvent résulter de la polymérisation de monomères à insaturation éthylénique ayant au moins un groupement acide et/ou des esters de ces monomères acides et/ou des amides de ces monomères acides.

Comme monomère porteur de groupement acide, on peut utiliser des acides carboxyliques insaturés α,β-éthyléniques tels que l'acide acrylique, l'acide méthacrylique, l'acide crotonique, l'acide maléique, l'acide itaconique. On utilise de préférence l'acide (méth)acrylique et l'acide crotonique, et plus préférentiellement l'acide (méth)acrylique.

Les esters de monomères acides sont avantageusement choisis parmi les esters de l'acide (méth)acrylique (encore appelé les (méth)acrylates), notamment des (méth)acrylates d'alkyle, en particulier d'alkyle en C₁-C₂₀, de préférence en C₁-C₈, des (méth)acrylates d'aryle, en particulier d'aryle en C₆-C₁₀, des (méth)acrylates d'hydroxyalkyle, en particulier d'hydroxyalkyle en C₂-C₆.
Parmi les (méth)acrylates d'alkyle, on peut citer le méthacrylate de méthyle, le méthacrylate d'éthyle, le méthacrylate de butyle, le méthacrylate d'isobutyle, le méthacrylate d'éthyl-2 hexyle, le méthacrylate de lauryle.
Parmi les (méth)acrylates d'hydroxyalkyle, on peut citer l'acrylate d'hydroxyéthyle, l'acrylate de 2-hydroxypropyle, le méthacrylate d'hydroxyéthyle, le méthacrylate de 2-hydroxypropyle.
Parmi les (méth)acrylates d'aryle, on peut citer l'acrylate de benzyle et l'acrylate de phényle.
Les esters de l'acide (méth)acrylique particulièrement préférés sont les (méth)acrylates d'alkyle.

Selon la présente invention, le groupement alkyle des esters peut être soit fluoré, soit perfluoré, c'est-à-dire qu'une partie ou la totalité des atomes d'hydrogène du groupement alkyle sont substitués par des atomes de fluor.

Comme amides des monomères acides, on peut par exemple citer les (méth)acrylamides, et notamment les N-alkyl (méth)acrylamides, en particulier d'alkyl en C₂-C₁₂. Parmi les N-alkyl (méth)acrylamides, on peut citer le N-éthyl acrylamide, le N-t-butyl acrylamide et le N-t-octyl acrylamide.

Les polymères filmogènes vinyliques peuvent également résulter de l'homopolymérisation ou de la copolymérisation de monomères choisis parmi les esters vinyliques et les monomères styrèniques. En particulier, ces monomères peuvent être polymérisés avec des monomères acides et/ou leurs esters et/ou leurs amides, tels que ceux mentionnés précédemment.
Comme exemple d'esters vinyliques, on peut citer l'acétate de vinyle, le néodécanoate de vinyle, le pivalate de vinyle, le benzoate de vinyle et le t-butyl benzoate de vinyle.
Comme monomères styrèniques, on peut citer le styrène et l'alpha-méthyl styrène.

La liste des monomères donnée n'est pas limitative et il est possible d'utiliser tout monomère connu de l'homme du métier entrant dans les catégories de monomères acryliques et vinyliques (y compris les monomères modifiés par une chaîne siliconée).

Comme polymère filmogène acrylique utilisable selon l'invention, on peut citer ceux vendus sous les dénominations NEOCRYL XK-90® , NEOCRYL A-1070® , NEOCRYL A-1090®, NEOCRYL BT-62®, NEOCRYL A-1079®, NEOCRYL A-523® par la société AVECIA-NEORESINS, DOW LATEX 432® par la société DOW CHEMICAL.

On peut aussi citer, parmi les polycondensats utilisables comme polymère filmogène, les polyuréthannes anioniques, cationiques, non-ioniques ou amphotères, les polyuréthannes-acryliques, les polyuréthannes-polyvinylpirrolidones, les polyester-polyuréthannes, les polyéther-polyuréthannes, les polyurées, les polyurée/polyuréthannes, et leurs mélanges.
Le polyuréthanne filmogène peut être, par exemple, un copolymère polyuréthanne, polyurée/uréthanne, ou polyurée, aliphatique, cycloaliphatique ou aromatique, comportant seule ou en mélange :
- au moins une séquence d'origine polyester aliphatique et/ou cycloaliphatique et/ou aromatique, et/ou,
- au moins une séquence siliconée, ramifiée ou non, par exemple polydiméthylsiloxane ou polyméthylphénylsiloxane, et/ou
- au moins une séquence comportant des groupes fluorés.

Les polyuréthannes filmogènes tels que définis dans l'invention peuvent être également obtenus à partir de polyesters, ramifiés ou non, ou d'alkydes comportant des hydrogènes mobiles que l'on modifie par réaction avec un diisocyanate et un composé organique bifonctionnel (par exemple dihydroxy, diamino ou hydroxyamino), comportant en plus soit un groupement acide carboxylique ou carboxylate, soit un groupement acide sulfonique ou sulfonate, soit encore un groupement amine tertiaire neutralisable ou un groupement ammonium quaternaire.

Comme polyuréthanne filmogène utilisable selon l'invention on peut utiliser ceux commercialisés sous les dénominations NEOREZ R-981®, NEOREZ R-974® par la société AVECIA-NEORESINS, les AVALURE UR-405®, AVALURE UR-410®, SANCURE 875®, SANCURE 2060®, AVALURE UR-425® , AVALURE UR-430®, SANCURE 861®, SANCURE 878®, AVALURE UR-450® par la société GOODRICH.

Parmi les polycondensats filmogènes, on peut également citer les polyesters, les polyesters amides, les polyesters à chaîne grasse, les polyamides, et les résines époxyesters.

Les polyesters peuvent être obtenus, de façon connue, par polycondensation d'acides dicarboxyliques avec des polyols, notamment des diols.
L'acide dicarboxylique peut être aliphatique, alicyclique ou aromatique. On peut citer comme exemple de tels acides : l'acide oxalique, l'acide malonique, l'acide diméthylmalonique, l'acide succinique, l'acide glutarique, l'acide adipique, l'acide pimélique, l'acide 2,2-diméthylglutarique, l'acide azélaïque, l'acide subérique, l'acide sébacique, l'acide fumarique, l'acide maléique, l'acide itaconique, l'acide phtalique, l'acide dodécanedioïque, l'acide 1,3-cyclohexanedicarboxylique, l'acide 1,4-cyclohexanedicarboxylique, l'acide isophtalique, l'acide téréphtalique, l'acide 2,5-norbornane dicarboxylique, l'acide diglycolique, l'acide thiodipropionique, l'acide 2,5-naphtalènedicarboxylique, l'acide 2,6-naphta-lènedicarboxylique. Ces monomères acide dicarboxylique peuvent être utilisés seuls ou en combinaison d'au moins deux monomères acide dicarboxylique. Parmi ces monomères, on choisit préférentiellement l'acide phtalique, l'acide isophtalique, l'acide téréphtalique.

Le diol peut être choisi parmi les diols aliphatiques, alicycliques, aromatiques. On utilise de préférence un diol choisi parmi : l'éthylène glycol, le diéthylène glycol, le triéthylène glycol, le 1,3-propanediol, le cyclohexane diméthanol, le 4-butanediol. Comme autres polyols, on peut utiliser le glycérol, le pentaérythritol, le sorbitol, le triméthylol propane.

Les polyesters amides peuvent être obtenus de manière analogue aux polyesters, par polycondensation de diacides avec des diamines ou des amino alcools. Comme diamine, on peut utiliser l'éthylènediamine, l'hexaméthylènediamine, la méta- ou para-phénylènediamine. Comme aminoalcool, on peut utiliser la monoéthanolamine.

Le polyester peut en outre comprendre au moins un monomère portant au moins un groupement -SO₃M, avec M représentant un atome d'hydrogène, un ion ammonium NH₄⁺ ou un ion métallique, comme par exemple un ion Na⁺, Li⁺, K+, Mg²⁺, Ca²⁺, Cu²⁺, Fe²⁺, Fe³⁺. On peut utiliser notamment un monomère aromatique bifonctionnel comportant un tel groupement -SO₃M.

Le noyau aromatique du monomère aromatique bifonctionnel portant en outre un groupement -SO₃M tel que décrit ci-dessus peut être choisi par exemple parmi les noyaux benzène, naphtalène, anthracène, diphényl, oxydiphényl, sulfonyldiphényl, méthylènediphényl. On peut citer comme exemple de monomère aromatique bifonctionnel portant en outre un groupement -SO₃M : l'acide sulfoisophtalique, l'acide sulfotéréphtalique, l'acide sulfophtalique, l'acide 4-sulfonaphtalène-2,7-dicarboxylique.
On préfère utiliser dans les compositions objet de l'invention des copolymères à base d'isophtalate/sulfoisophtalate, et plus particulièrement des copolymères obtenus par condensation de di-éthylèneglycol, cyclohexane di-méthanol, acide isophtalique, acide sulfoisophtalique. De tels polymères sont vendus par exemple sous le nom de marque Eastman AQ par la société Eastman Chemical Products.

Les polymères d'origine naturelle, éventuellement modifiés, peuvent être choisis parmi la résine shellac, la gomme de sandaraque, les dammars, les élémis, les copals, les polymères cellulosiques insolubles dans l'eau, et leurs mélanges.

On peut encore citer les polymères résultant de la polymérisation radicalaire d'un ou plusieurs monomères radicalaires à l'intérieur et/ou partiellement en surface, de particules préexistantes d'au moins un polymère choisi dans le groupe constitué par les polyuréthannes, les polyurées, les polyesters, les polyesteramides et/ou les alkydes. Ces polymères sont généralement appelés polymères hybrides.

La dispersion comprenant un ou plusieurs polymères filmogènes peut être préparée par l'homme du métier sur base de ses connaissances générales.

La taille des particules de polymère filmogène en dispersion aqueuse peut aller de 10 à 500 nm, et va de préférence de 20 à 300 nm.

Le polymère filmogène peut être présent dans la composition selon l'invention en une teneur allant de 1 % à 50 % en poids, de préférence de 5 % à 40% en poids, de matière sèche de polymères filmogènes par rapport au poids total de la composition.

Avantageusement, le polymère filmogène et le polymère superabsorbant d'eau peuvent être présents dans la composition selon l'invention en un rapport pondéral polymère / polymère allant de 5 à 15.

La composition selon l'invention peut comprendre un agent auxiliaire de filmification favorisant la formation d'un film avec les particules du polymère filmogène. Un tel agent de filmification peut être choisi parmi tous les composés connus de l'homme du métier comme étant susceptibles de remplir la fonction recherchée, et notamment être choisi parmi les agents plastifiants et les agents de coalescence.

Le milieu de la composition de l'invention est avantageusement un milieu aqueux contenant de l'eau. La teneur en eau dans la composition peut aller de 3 % à 98,9 % en poids, par rapport au poids total de la composition, et mieux de 10 % à 90 % en poids.

La composition peut comprendre en outre un agent épaississant. Parmi les agents épaississants utilisables selon l'invention, on peut citer :
- les épaississants cellulosiques hydrosoluble tels que l'hydroxyéthylcellulose, la méthylcellulose, l'hydroxypropylcellulose. Parmi celles-ci, on peut citer notamment les gommes vendues sous la dénomination de "Cellosize QP 44001 H" par la Société Amercol,
- la gomme de guar, notamment celles vendues sous la dénomination VIDOGUM GH 175 par la société UNIPECTINE et sous la dénomination JAGUAR C par la société MEYHALL,
- la gomme de guar quaternisée vendue sous la dénomination de "Jaguar C-13-S" par la Société Meyhall,
- les gommes de guar non-ioniques comprenant des groupements hydroxyalkyle en C₁-C₆. On peut mentionner à titre d'exemple, les groupements hydroxyméthyle, hydroxyéthyle, hydroxypropyle et hydroxybutyle. De telles gommes de guar sont notamment vendues sous les dénominations commerciales JAGUAR HP8, JAGUAR HP60 et JAGUAR HP120, JAGUAR DC 293 et JAGUAR HP 105 par la société MEYHALL, ou sous la dénomination GALACTASOL 4H4FD2 par la société AQUALON.
- les gommes de xanthane, de caroube, de scléroglucane, de gellane, de rhamsan, de karoya,
- les alginates, la maltodextrine, l'amidon et ses dérivés, l'acide hyaluronique et ses sels,
- les argiles, et notamment les montmorillonites, les hectorites, les laponites
- les acides polyacryliques réticulés tels que les "Carbopol" de la Société Goodrich,
- les polymères poly(métha)crylates de glycéryle vendus sous les dénominations de "Hispagel" ou "Lubragel" par les Sociétés Hispano Quimica ou Guardian,
- la polyvinylpyrrolidone,
- l'alcool polyvinylique,
- les polymères et les copolymères réticulés d'acrylamide, tels que ceux vendus sous les dénominations de "PAS 5161" ou "Bozepol C" par la Société Hoechst, de "Sepigel 305" par la Société Seppic, ou encore
- les homopolymères réticulés de chlorure de méthacryloyloxyéthyltriméthylammonium vendus sous la dénomination de "Salcare SC95" par la Société Allied Colloïd.
- les polymères associatifs et notamment les polyuréthanes associatifs.

Dans la composition selon l'invention, l'agent épaississant peut être présent en une teneur allant de 0,1 % à 20 % en poids, par rapport au poids total de la composition, et de préférence de 1 % à 10 % en poids.

Pour favoriser le séchage rapide de la composition après son application sur la peau et/ou les phanères, la composition peut comprendre des accélérateurs de séchage comme les solvants volatils et de préférence des solvants organiques volatils miscibles à l'eau, comme l'éthanol. La quantité de tels solvants organiques est choisie de telle sorte que la viscosité de la composition soit bien maintenue dans la gamme définie précédemment. Ces solvants organiques peuvent être présent dans la composition en une teneur allant jusqu'à 15 % en poids, par rapport au poids total de la composition, (notamment de 0,1 % à 15 %) et de préférence jusqu'à 10 % en poids (notamment de 0,5 % à 10 %). Par volatil, on entend un composé apte à s'évaporer au contact de la peau, à température ambiante.

La composition peut comprendre également un colorant hydrosoluble. En particulier, il est possible d'ajouter un colorant hydrosoluble dans l'eau employée pour faire gonfler le polymère superabsorbant, ce qui permet d'obtenir des cristaux colorés rendant un bel aspect de bijoux.

Parmi les colorants hydrosolubles, on peut citer le sel disodique de ponceau, le sel disodique du vert d'alizarine, le sel disodique du bleu brillant, le jaune de quinoléine, le sel trisodique d'amarante, le sel disodique de tartrazine, le sel monosodique de rhodamine, le sel disodique de fuchsine, la xanthophylle, leurs mélanges.

La composition peut en outre comprendre d'autres ingrédients habituellement utilisés en cosmétiques. De tels ingrédients peuvent être notamment des agents plastifiants, des agents de coalescence, des charges, des matières colorantes comme les pigments ou les colorants, des cires, des tensio-actifs, des conservateurs, des huiles, des agents hydratants, des parfums qui sont bien connus de l'état de la technique. Bien entendu, l'homme du métier veillera à choisir ce ou ces éventuels additifs et/ou leur quantité, de manière telle que les propriétés avantageuses de dépôt tridimensionnel sur la peau et/ou les phanères soient conservées.

La composition peut être appliquée sur la peau à l'aide de tout applicateur connu de l'homme du métier tels que les brosses, les applicateurs à embout mousse, à plume floquée, en feutre, les pinceaux, les spatules.

La composition selon l'invention peut être également appliquée sur la peau à l'aide d'un pochoir : la composition ainsi déposée dans les parties évidées du pochoir conserve sa forme après le retrait du pochoir, donnant ainsi un maquillage sur la peau très décoratif.

L'invention est illustrée plus en détail dans l'exemple suivant.

### Exemple 1 :

On a préparé une composition de maquillage de la peau comprenant les ingrédients suivants :
- Polymère superabsorbant d'eau vendu sous la dénomination "SALSORB CL 10" par la société ALLIED COLLOIDS 2,5 g
- Polyuréthane en dispersion aqueuse à 35 % de polymère ("AVALURE UR-405" de la société GOODRICH) 21 g MA
- Sel disodique de bleu brillant (colorant bleu) 0,001 g
- Conservateur qs
- Eau qsp 100 g

La composition a été préparée, à température ambiante, en faisant gonfler le polymère superabsorbant d'eau dans l'eau additionnée du colorant et du conservateur puis on a ajouté à ce mélange le polyuréthane en dispersion aqueuse.

On a obtenu une composition de maquillage contenant des particules de polymère superabsorbant d'eau gonflées d'eau présentant une taille de particules allant de 2 à 5 mm.
Après application de la composition sur la peau, on obtient un maquillage présentant l'aspect de pierres transparentes bleutées.
Cette composition appliquée sur les cils permet d'épaissir ces derniers.

## Revendications

1. Composition cosmétique comprenant, dans un milieu cosmétiquement acceptable,un polymère filmogène et un polymère superabsorbant d'eau sous forme de particules gonflées d'eau ayant une taille moyenne au moins égale à 0,5 mm.

2. Composition selon la revendication 1, **caractérisée par le fait que** le polymère superabsorbant d'eau est apte à absorber au moins 20 g d'eau par gramme dudit polymère.

3. Composition selon la revendication 1 ou 2, **caractérisée par le fait que** polymère superabsorbant d'eau peut absorber de 20 à 2000 g d'eau par gramme dudit polymère.

4. Composition selon l'une quelconque des revendications précédentes, **caractérisée par le fait que** la taille moyenne des particules gonflées d'eau du polymère absorbant d'eau va de 0,5 mm à 20 mm, de préférence de 1 mm à 15 mm, et mieux de 3 à 10 mm.

5. Composition selon l'une quelconque des revendications précédentes, **caractérisée par le fait que** le polymère superabsorbant d'eau est choisi dans le groupe formé par :
- les polymères résultants de la polymérisation avec réticulation partielle de monomères à insaturation éthylénique hydrosolubles,
- les amidons greffés polyacrylates,
- les copolymères acrylamide/acide acrylique, notamment sous forme de sels de sodium,
- les amidons greffés acrylamide/acide acrylique, notamment sous forme de sels de sodium ou de potassium,
- les copolymères isobutylène/anhydride maléique,
- les sels de sodium ou de potassium de carboxyméthyl cellulose,
- les sels d'acide polyaspartique réticulés,
- les associations chitosane/polyvinyl pyrrolidone, chitosane /polyéthylène imine.

6. Composition selon l'une quelconque des revendications précédentes, **caractérisée par le fait que** le polymère absorbant d'eau est un polyacrylate de sodium réticulé.

7. Composition selon l'une quelconque des revendications précédentes, **caractérisée par le fait que** le polymère est présent en une teneur allant de 0,1 % à 50 % en poids, par rapport au poids total de la composition, et de préférence de 0,5 % à 40 % en poids.

8. Composition selon l'une quelconque des revendications précédentes, **caractérisée par le fait que** le polymère filmogène est choisi dans le groupe formé par les polymères radicalaires, les polycondensats, les polymères d'origine naturelle et leurs mélanges.

9. Composition selon l'une quelconque des revendications précédentes, **caractérisée par le fait que** le polymère filmogène est choisi dans le groupe formé par les polymères vinyliques résultant de la polymérisation de monomères choisis parmi les acides carboxyliques insaturés α,β-éthyléniques, les esters de cesdits acides, les amides de cesdits acides, les esters vinyliques, les monomères styréniques.

10. Composition selon l'une quelconque des revendications précédentes, **caractérisée par le fait que** le polymère filmogène est choisi dans le groupe formé par les polyuréthanes, les polyesters, les polyesters amides, les polyamides, les résines époxyesters.

11. Composition selon l'une quelconque des revendications précédentes, **caractérisée par le fait que** le polymère filmogène est choisi dans le groupe formé par par la résine shellac, la gomme de sandaraque, les dammars, les élémis, les copals, les polymères cellulosiques insolubles dans l'eau, et leurs mélanges.

12. Composition selon l'une quelconque des revendications précédentes, **caractérisée par le fait que** le polymère filmogène se présente sous la forme de dispersion aqueuse de particules.

13. Composition selon l'une quelconque des revendications précédentes, **caractérisée par le fait que** le polymère filmogène est présent en une teneur allant de 1 % à 50 % en poids, par rapport au poids total de la composition.

14. Composition selon l'une quelconque des revendications précédentes, **caractérisée par le fait qu'**elle comprend un colorant hydrosoluble.

15. Composition selon l'une quelconque des revendications précédentes, **caractérisée par le fait qu'**elle comprend un agent épaississant.

16. Composition selon l'une quelconque des revendications précédentes, **caractérisée par le fait qu'**elle comprend, en outre, au moins un additif choisi dans le groupe formé par les agents plastifiants, les agents de coalescence, les charges, les matières colorantes, les cires, les tensio-actifs, les conservateurs, les huiles, les agents hydratants, les parfums, et leurs mélanges.

17. Procédé de maquillage des matières kératiniques comprenant l'application sur lesdites matières kératiniques d'une composition selon l'une des revendications 1 à 16.

18. Utilisation d'une composition selon l'une quelconque des revendications 1 à 16, pour obtenir un maquillage des matières kératiniques présentant l'aspect de cristaux et/ou de bijou.

19. Utilisation, dans une composition cosmétique, d'un polymère filmogène et de particules d'un polymère superabsorbant d'eau sous forme de particules gonflée d'eau ayant une taille moyenne au moins égale à 0,5 mm, pour obtenir un maquillage des matières kératiniques présentant l'aspect de cristaux et/ou d'un bijou.

20. Utilisation d'une composition selon l'une quelconque des revendications 1 à 16, pour épaissir les cils.

21. Utilisation, dans une composition cosmétique, d'un polymère filmogène et de particules d'un polymère superabsorbant d'eau pour épaissir les cils.

## Patentansprüche

1. Kosmetische Zusammensetzung, die in einem kosmetisch akzeptablen Medium ein filmbildendes Polymer und ein Wasser superabsorbierendes Polymer in Form von in Wasser gequollenen Partikeln mit einer mittleren Größe von mindestens 0,5 mm enthält.

2. Zusammensetzung nach Anspruch 1, **dadurch gekennzeichnet, dass** das Wasser superabsorbierende Polymer befähigt ist, mindestens 20 g Wasser auf 1 g Polymer zu absorbieren.

3. Zusammensetzung nach Anspruch 1 oder 2, **dadurch gekennzeichnet, dass** das Wasser superabsorbierende Polymer 20 bis 2 000 g Wasser auf 1 g Polymer absorbieren kann.

4. Zusammensetzung nach einem der vorhergehenden Ansprüche, **dadurch gekennzeichnet, dass** die mittlere Größe der in Wasser gequollenen Partikel des Wasser absorbierenden Polymers im Bereich 0,5 bis 20 mm, vorzugsweise 1 bis 15 mm und besser 3 bis 10 mm liegt.

5. Zusammensetzung nach einem der vorhergehenden Ansprüche, **dadurch gekennzeichnet, dass** das Wasser superabsorbierende Polymer ausgewählt ist unter:
- Polymeren, die bei der Polymerisation von wasserlöslichen Monomeren mit ethylenischer Doppelbindung unter teilweiser Vernetzung erhalten werden,
- mit Stärke gepfropften Polyacrylaten,
- Acrylamid/Acrylsäure-Copolymeren, insbesondere in Form der Natriumsalzen,
- mit Stärke gepfropften Acrylamid/Acrylsäure-Polymeren, insbesondere in Form der Natrium- oder Kaliumsalze,
- Isobutylen/Maleinsäureanhydrid-Copolymeren,
- Natrium- oder Kaliumsalzen von Carboxymethylcellulose,
- vernetzten Polyasparaginsäuresalzen, und
- den Kombinationen Chitosan/Polyvinylpyrrolidon und Chitosan/ Polyethylenimin.

6. Zusammensetzung nach einem der vorhergehenden Ansprüche, **dadurch gekennzeichnet, dass** das Wasser absorbierende Polymer ein vernetztes Natriumpolyacrylat ist.

7. Zusammensetzung nach einem der vorhergehenden Ansprüche, **dadurch gekennzeichnet, dass** das Polymer in einer Menge von 0,1 bis 50 Gew.-%, bezogen auf das Gesamtgewicht der Zusammensetzung, und vorzugsweise 0,5 bis 40 Gew.-% enthalten ist.

8. Zusammensetzung nach einem der vorhergehenden Ansprüche, **dadurch gekennzeichnet, dass** das filmbildende Polymer unter den durch radikalische Polymerisation hergestellten Polymeren, Polykondensaten, Polymeren natürlicher Herkunft und deren Gemischen ausgewählt ist.

9. Zusammensetzung nach einem der vorhergehenden Ansprüche, **dadurch gekennzeichnet, dass** das filmbildende Polymer ausgewählt ist unter Vinylpolymeren, die bei der Polymerisation von Monomeren entstehen, die unter den α,β-ethylenisch ungesättigten Carbonsäuren, den Estern dieser Säuren, den Amiden dieser Säuren, wie Vinylestern und Styrolmonomeren ausgewählt sind.

10. Zusammensetzung nach einem der vorhergehenden Ansprüche, **dadurch gekennzeichnet, dass** das filmbildende Polymer unter den Polyurethanen, Polyestern, Polyesteramiden, Polymiden und Epoxyesterharzen ausgewählt ist.

11. Zusammensetzung nach einem der vorhergehenden Ansprüche, **dadurch gekennzeichnet, dass** das filmbildende Polyme unter Schellak, Sandarak, Dammarharzen, Elemi, Kopalen und in Wasser unlöslichen Cellulosepolymeren und deren Gemischen ausgewählt ist.

12. Zusammensetzung nach einem der vorhergehenden Ansprüche, **dadurch gekennzeichnet, dass** das filmbildende Polymer in Form einer wässrigen Dispersion von Partikeln vorliegt.

13. Zusammensetzung nach einem der vorhergehenden Ansprüche, **dadurch gekennzeichnet, dass** das filmbildende Polymer in einer Menge von 1 bis 50 Gew.-%, bezogen auf das Gesamtgewicht der Zusammensetzung, enthalten ist.

14. Zusammensetzung nach einem der vorhergehenden Ansprüche, **dadurch gekennzeichnet, dass** sie einen wasserlöslichen Farbstoff enthält.

15. Zusammensetzung nach einem der vorhergehenden Ansprüche, **dadurch gekennzeichnet, dass** sie ein Verdickungsmittel enthält.

16. Zusammensetzung nach einem der vorhergehenden Ansprüche, **dadurch gekennzeichnet, dass** sie ferner mindestens einen Zusatzstoff enthält, der unter den Weichmachern, Koaleszenzmitteln, Füllstoffen, Farbmitteln, Wachsen, grenzflächenaktiven Stoffen, Konservierungsmitteln, Ölen, Hydratisierungsmitteln, Parfums und deren Gemischen ausgewählt ist.

17. Verfahren zum Schminken von Keratinsubstanzen, das das Aufbringen einer Zusammensetzung nach einem der Ansprüche 1 bis 16 auf die Keratinsubstanzen umfasst.

18. Verwendung einer Zusammensetzung nach einem der Ansprüche 1 bis 16 zur Bildung einer Schminke auf Keratinsubstanzen, die das Aussehen von Kristallen und/oder Schmuck hat.

19. Verwendung eines filmbildenden Polymers und von Partikeln eines Wasser superabsorbierenden Polymers in Form von in Wasser gequollenen Partikeln mit einer mittleren Größe von mindestens 0,5 mm in einer kosmetischen Zusammensetzung, um eine Schminke auf Keratinsubstanzen zu erzeugen, die das Aussehen von Kristallen und/oder Schmuck hat.

20. Verwendung einer Zusammensetzung nach einem der Ansprüche 1 bis 16, um Wimpern dichter erscheinen zu lassen.

21. Verwendung eines filmbildenden Polymers und von Partikeln eines Wasser superabsorbierenden Polymers in einer kosmetischen Zusammensetzung, um Wimpern dichter zu machen.

## Claims

1. Cosmetic composition comprising, in a cosmetically acceptable medium, a film-forming polymer and a water-superabsorbent polymer in the form of water-swollen particles with a mean size of at least 0.5 mm.

2. Composition according to Claim 1, **characterized in that** the water-superabsorbent polymer is capable of absorbing at least 20 g of water per gram of the said polymer.

3. Composition according to Claim 1 or 2, **characterized in that** the water-superabsorbent polymer can absorb 20 to 2000 g of water per gram of the said polymer.

4. Composition according to any one of the preceding claims, **characterized in that** the mean size of the water-swollen particles of the water-absorbing polymer ranges from 0.5 mm to 20 mm, preferably from 1 mm to 15 mm and better still from 3 to 10 mm.

5. Composition according to any one of the preceding claims, **characterized in that** the water-superabsorbent polymer is chosen from the group formed by:
- polymers resulting from the polymerization with partial crosslinking of ethylenically unsaturated water-soluble monomers,
- polyacrylate-grafted starches,
- acrylamide/acrylic acid copolymers, especially in the form of sodium salts,
- acrylamide/acrylic acid-grafted starches, especially in the form of sodium or potassium salts,
- isobutylene/maleic anhydride copolymers,
- sodium or potassium salts of carboxymethyl cellulose,
- crosslinked polyaspartic acid salts,
- chitosan/polyvinylpyrrolidone or chitosan/polyethyleneimine combinations.

6. Composition according to any one of the preceding claims, **characterized in that** the water-absorbing polymer is a crosslinked sodium polyacrylate.

7. Composition according to any one of the preceding claims, **characterized in that** the polymer is present in a content ranging from 0.1% to 50% by weight and preferably from 0.5% to 40% by weight relative to the total weight of the composition.

8. Composition according to any one of the preceding claims, **characterized in that** the film-forming polymer is chosen from the group formed by free-radical polymers, polycondensates and polymers of natural origin, and mixtures thereof.

9. Composition according to any one of the preceding claims, **characterized in that** the film-forming polymer is chosen from the group formed by vinyl polymers resulting from the polymerization of monomers chosen from α,β-ethylenically unsaturated carboxylic acids, the esters of the said acids, the amides of the said acids, vinyl esters and styrene monomers.

10. Composition according to any one of the preceding claims, **characterized in that** the film-forming polymer is chosen from the group formed by polyurethanes, polyesters, polyesteramides, polyamides and epoxyester resins.

11. Composition according to any one of the preceding claims, **characterized in that** the film-forming polymer is chosen from the group formed by shellac resin, sandarac gum, dammar resin, elemi gum, copal resin and water-insoluble cellulose-based polymers, and mixtures thereof.

12. Composition according to any one of the preceding claims, **characterized in that** the film-forming polymer is in the form of an aqueous particle dispersion.

13. Composition according to any one of the preceding claims, **characterized in that** the film-forming polymer is present in a content ranging from 1% to 50% by weight relative to the total weight of the composition.

14. Composition according to any one of the preceding claims, **characterized in that** it comprises a water-soluble dye.

15. Composition according to any one of the preceding claims, **characterized in that** it comprises a thickener.

16. Composition according to any one of the preceding claims, **characterized in that** it also comprises at least one additive chosen from the group formed by plasticizers, coalescers, fillers, dyestuffs, waxes, surfactants, preserving agents, oils, moisturizers and fragrances, and mixtures thereof.

17. Process for making up keratin materials, comprising the application to the said keratin materials of a composition according to one of Claims 1 to 16.

18. Use of a composition according to any one of Claims 1 to 16, for obtaining makeup on keratin materials that has the appearance of crystals and/or a jewel.

19. Use, in a cosmetic composition, of a film-forming polymer and of particles of a water-superabsorbent polymer in the form of water-swollen particles with a mean size of at least 0.5 mm, for obtaining makeup on keratin materials that has the appearance of crystals and/or a jewel.

20. Use of a composition according to any one of Claims 1 to 16, for thickening the eyelashes.

21. Use, in a cosmetic composition, of a film-forming polymer and of particles of a water-superabsorbent polymer, for thickening the eyelashes.
